# EUROPEAN PATENT SPECIFICATION

(11) **EP 0 817 815 B1**
(45) Date of publication and mention of the grant of the patent: **05.07.2000**
(21) Application number: 96907557.1
(22) Date of filing: 18.03.1996
(51) Int. Cl.: C09J 133/00, C09J 167/00, C09J 183/00, C09J 175/00, A61L 15/58, G03F 7/038

(54) **ADHESIVES**
KLEBSTOFFE
ADHESIFS

(30) Priority: 18.03.1995 GB 9505495
(43) Date of publication of application: 14.01.1998
(73) Proprietor: Smith & Nephew PLC, London WC2N 6LA (GB)
(72) Inventor: WEBSTER, Iain, Osbaldwick, York YO1 3PG (GB)
(86) International application number: GB9600630
(87) International publication number: WO9629374

(56) References cited:
- US-A- 2 980 535
- US-A- 3 453 110
- US-A- 3 655 625
- US-A- 3 737 407
- DATABASE WPI Section Ch, Week 9424 Derwent Publications Ltd., London, GB; Class A81, AN 94-197360 XP002006112 & JP,A,06 136 326 (TOPCON CORP) , 17 May 1994
- DATABASE WPI Section Ch, Week 9441 Derwent Publications Ltd., London, GB; Class A23, AN 94-330150 XP002006113 & JP,A,06 256 494 (MITSUBISHI GAS CHEM CO INC) , 13 September 1994

## Description

The present invention relates to pressure sensitive adhesives, methods for their preparation and to articles comprising pressure sensitive adhesives.

Adhesive products, such as adhesive surgical or medical dressings, normally comprise a layer of a pressure sensitive adhesive. Such a layer of adhesive is commonly provided by coating a solution of the adhesive in a volatile organic solvent onto a suitable substrate and drying the coating in a heated oven to remove the solvent. Solution coating of adhesives, however, can be hazardous due to the flammability or toxicity of the solvents, such as toluene, which are commonly used in adhesive coating solutions. Furthermore, an adhesive solution coating process in which the solvent is not recovered or in which the coated adhesive layer is a relatively thick layer and requires a long drying time may be relatively uneconomic.

A solventless coating process of providing an adhesive layer would therefore be an advantage in mitigating the abovementioned disadvantages as well as being more environmentally friendly.

It is known that liquid oligomer compositions containing unsaturated end groups can be coated on a substrate and cured rapidly by radiation such as electron beam or ultra violet light radiation to form adhesive layers. United States Patent No.4022926 discloses a method of making adhesive labels in which the adhesive and base layers are formed by radiation curing. The Patent further discloses that curable compositions for both layers preferably comprise a polyurethane capped with residues of a hydroxy alkyl acrylate or methacrylate. The curable composition for the adhesive layer is prepared by adding tackifying resins or agents to the curable composition used for the base layer.

US Patent No.2980535 describes light sensitive photographic elements comprising coated with a material consisting of a polymer, eg. cellulose with a formyl derivative, eg. 9-anthracene aldehyde.

Similarly, US Patent No.3453110 describes a polymer, eg. a cellulose carrying coumarin substituents. Such materials are not known to possess adhesive properties.

US Patent Nos.3655625 and 3737407 both describe light sensitive polymers for forming lithographic plates and etching resists. The polymers of US3655625 generally comprise a polyurethane resin which is heated with a cinnamoylating agent to form a photographer, whereas US3737407 describes bisphenol-A-fumarate polyester cinnamate polymer. Such materials are not known to be suitable as adhesives.

JP-A-06136326 describes adhesives which comprise; for example, diethylene glycol diallyl carbonate with a cinnamic acid. The ingredients are photosensitive to form the adhesive but are not known to be photo switchable.

JP-A-06256494 describes polycarbonate resins which comprise a polycarbonate terminated by a chalcone derivative. The object of the invention is to produce a polycarbonate which does not hydrolyse and which can absorb UV light. However, the chalcone derivatives are not known to dimerise such that the structure and strength of the polycarbonate is affected.

European Patent No.EP-A-0 282 554 discloses skin-friendly, inherently tacky polyurethane gel or foam adhesives, suitable for woundcare applications and containing reduced amounts of leachable materials. Polyurethane-acrylate adhesives are disclosed which may be prepared by reacting an isocyanate capped prepolymer with one or more polyols or water and optionally with various mono-ols, one of which contains an unsaturated functionality which is reactive when exposed to irradiation such as UV or electron beam irradiation. The unsaturated functionality is exposed to UV light to produce cross-links in the adhesive before the adhesive is ready to use.

The abovementioned adhesives are however disadvantageous in that they still require the use of photoinitiators which lead to toxicity problems and a greater chance of migratable materials, eg. leachable materials, in the final product. It is also known that free-radical reactions can be prone to oxygen inhibition. Furthermore, free-radical initiation can add to the expense and to the time required to prepare polyurethane adhesives and free-radical reactions also suffer from the disadvantage that they are difficult to control and can be adversely affected by the presence of even low levels of impurities.

European Patent Application No.EP-A-0 448 741 describes a solventless adhesive system which is a pressure sensitive adhesive comprising a polyacrylate backbone cross-linked with polymerised benzophenone units.

The present invention aims to provide UV curable adhesives formed by a method which does not require the use of free-radical reactions and which contains low or neglible levels of leachable materials.

We have now surprisingly found that pressure sensitive adhesives may be manufactured comprising a polymeric backbone and a dimerised capping group.

Thus according to the invention we provide a pressure sensitive adhesive obtainable by photodimerising prepolymer molecules comprising a polymeric backbone capped with a dimerisable group.

Suitable backbone polymers include such polymeric chains conventionally used in adhesive systems and especially those selected from polyurethanes, polyacrylates, silicones and polyesters. Of these polyurethanes and polyacrylates are particularly suitable for use in the pressure sensitive adhesives of the invention and especially preferred are polyacrylates. Any conventionally known polyacrylates may be used.

The polymeric molecules may be homopolymers or copolymers.

The dimerised groups may be present as end groups on the polymeric backbone, thus a linear pressure sensitive adhesive will be formed. However, it is not essential that the dimerised groups be incorporated as end groups since they may be present as side chains along the polymeric backbone. Thus, in such cases the pressure sensitive adhesive would comprise a cross-linked polymeric material. Although the pressure sensitive adhesive material may comprise linear or cross-linked polymers, substantially cross-linked polymers are preferred.

The dimerisable groups are preferably photodimerisable, eg. by UV light. The term "photo-dimerisable group" when used herein means a group capable of a cyclo-addition reaction when photochemically excited. The group may comprise a double bond or another unsaturated moiety.

Desirably the polymeric backbone molecules do not contain groups which form free radicals under conditions causing dimerisation of the dimerisable groups. Thus for example, unreacted acrylate or methacrylate groups are preferably not present.

The dimer comprises any suitable group, eg. an anthracene group, a cinnamyl group, a maleimide group, a coumarin group or a chalcone group. Especially preferred dimers are anthracene groups, eg. anthracene methyl groups.

Preferred dimerisable groups are mono-alcohols for reacting with the polymeric backbone to form a prepolymer are anthracene alcohols (eg. anthracene methanol), cinammyl alcohols and hydroxy maleimides. Especially preferred are anthracene alcohols, eg. anthracene methanol.

These groups may be optionally substituted with one or more substituents which do not substantially affect photo-dimerisation.

When the dimerisable group is reacting with the polymeric backbone a precursor to the pressure sensitive adhesive of the invention is formed, ie. such precursors can be cured to form a pressure sensitive adhesive. Such precursors are hereinafter referred to as prepolymers (although they are themselves polymeric in nature) since they are curable to form polymeric adhesives. The prepolymer molecules can be photo-dimerised to form any desirable polymer suitable for use in pressure sensitive adhesives. The prepolymers are themselves novel per se.

We also provide a precursor to a pressure sensitive adhesive as hereinbefore described which comprises a polymeric backbone capped with a dimerisable group.

The UV light used in curing the prepolymers of the present invention may be that conventionally known, for example, UV light of from 300 to 450nm wavelength. A typical intensity of UV irradiation is about 30mW cm⁻².

A further aspect of the invention is a prepolymer which comprises a polyacrylate backbone capped with anthracene alcohol groups, eg. anthracene methanol.

The polyurethane prepolymers aptly have a number average molecular weight of between 3000 and 15000 (molecular weights when referred to herein are as determined by gel permeation chromatography using polystyrene standards). The polyacrylate prepolymers aptly have a number average molecular weight of from 50,000 to 200,000, preferably from 50,000 to 150,000, eg. about 100,000.

Suitably polyurethane backbones for use in the present invention can be derived from a polyester diol and a di-isocyanate or, more preferably, from a polyether diol and a di-isocyanate.

Suitable polyether diols include polyoxyalkylene diols in which the alkylene contains 2 to 4 carbon atoms, such as polyoxyethylene, polyoxypropylene and polyoxytetramethylene diols and mixtures thereof. Such polyether diols can suitably have an average molecular weight of 1000 to 8000 and preferably have a molecular weight of 1500 to 6000. A favoured polyether diol for forming the polyurethanes used in the invention is polyoxypropylene diol. An apt diol of this type is known as PPG 2025, available from British Drug House, which has an average molecular weight of 2025. Another hydrophilic group containing diol is a block copolymer of polypropylene glycol and ethylene oxide marketed as Dowfax 63N10 (Trade Mark) available from Dow Chemicals Inc.

Polyoxyalkylene diol residues can be used to render the adhesive formed therefrom moisture vapour transmitting suitably have an isocyanate functionality of 1.6 - 2.05 and can preferably have an isocyanate functionality of 2.0. Suitable diisocyanates include aliphatic (including alicyclic) and aromatic diisocyanates.

Favoured diisocyanates include toluene diisocyanate, 4,4'-diphenylmethane diisocyanate and 4,4'-dicyclo hexyl diisocyanate, which is the preferred diisocyanate and which in an apt form is known as Desmodur W (Trade Mark), available from Bayer.

The polyurethane prepolymer can optionally be chain extended by a chain extending agent. Suitable chain extending agents, eg. for use with a polyurethane prepolymer include diols such as ethane diol and butane diol, diamines, for example ethylene diamine, and water.

The molar ratio of diol residues or diamine residues of diol and diamine residues (if both are present) to diisocyanate residues in the polyurethane backbone can suitably be 0.4 to 0.1:1 and is preferably 0.65 to 0.75:1, for example 0.7:1. This allows there to be an excess of diisocyanate molecules so that most polymeric backbone is isocyanate terminated.

Photo-dimerisable groups can then be incorporated by reacting the isocyanate terminated prepolymers with compounds comprising an isocyanate reactive group (eg. a hydroxy group or an amine group) and a dimerisable group. Preferably the compounds comprising the isocyanate reactive group are monofunctional in that they contain only a single isocyanate reactive group. More preferably, they are mono-alcohols.

Other compounds incorporating an isocyanate reactive group may be used to react with the polymeric backbone. Such compounds can include tackifying agents. These are also preferably mono-alcohols. Such mono-alcohols include mono hydroxy tackifying resins, eg. hydrogenated abietyl alcohol.

Preferably the isocyanate reactive compounds comprising dimerisable groups are used to react with from 10% to 28% of the isocyanate groups present, more preferably with from 13% to 22% of such groups.

The polyacrylates, silicones and polyesters used in the present invention may comrpise those conventionally known. Preferred polyacrylates are those described in European Patent No. EP-A-0 448 741 and especially British Patent No.2070631. Thus, preferred acrylates include, for example, alkyl acrylates such as n-butyl acrylates and hexyl acrylates such as 2-ethylhexyl acrylates. Especially preferred polyacrylates are those which include, for example, acrylic acid residues.

Preferred polyacrylates comprise, for example, from 16 to 62% n-butyl acrylate residues, 80 to 34%, 2-ethylhexyl acrylates residues and 4 to 10% acrylic acid residues. Further preferred polyacrylates are those described in British Patent No.2070631, which are incorporated herein by reference.

Adhesives according to the present invention can be used for a wide variety of purposes, eg. for securing industrial components together (such as components for use in electronics devices), or in the field of medicine.

Preferred adhesives are suitable for applying a medical dressing to a wound and should therefore not be cytotoxic or excessively strong adhesives.

The present invention therefore includes a medical dressing comprising an adhesive as hereinbefore described. The medical dressing may be, eg. a wound dressing, a pressure-relieving dressing, a catheter or cannula dressing, a bandage or a pad. The adhesive may also be used in or on other medical articles, eg. securing means (such as tapes etc) or prosthetic devices.

We also provide the use of an adhesive as hereinbefore described in the manufacture of a dressing.

Many medicinal agents may be incorporated into the adhesive of the present invention. By medicinal agent is meant pharmacologically active agents including agents which are topical anaesthetics such as xylocaine, bacteriostatic agents such as silver nitrate; anti-bacterial agents of which preferred forms are silver sulphadiazine and chlorhexidine salts; antibiotics; topical steroids, enzymes; tissue stimulants; coagulants and anticoagulants and antifungal agents. Other agents such as emollients may also be added.

According to the present invention, there is provided a method for forming an adhesive of the invention which comprises curing a prepolymer as hereinbefore described.

The prepolymers of the present invention may be produced using conventional processes known per se.

The present invention will now be described, without limitation thereof, by way of example only.

The following Trade Marks are referred to herein:

| **Trade Mark** | **Chemical/Apparatus** | **Company** |
|---|---|---|
| | | |
| Desmodur W | 4,4'-dicyclohexyl diisocyanate | Bayer UK Ltd |
| Metatin 812ES | Dioctyl tin dilaurate | Acima Chemical Industries Ltd Inc |
| Abitol | Hydroabietyl Alcohol | Hercules Incorporated |
| Melinex | Grade 542 polyethyleneterephthalate | ICI Films |
| Uvasol 400 | UV Cabinet | Dr Hönle |

### Example 1

### Preparation of Polyurethane Prepolymer

1.60g of polyethylene glycol (PEG) 600 was placed in an oven at 90°C for 15 mins to aid handling. 28.38g of polypropylene glycol (PPG) 2025, the melted PEG 600, 0.45 of Ethanediol (EDO) and 10.91 g of Desmodur W were added to a 250ml flange flask. The flask was placed in a water-bath at 90°C and the contents stirred to achieve a homogeneous mixture. This having been achieved, 0.1 g of Metatin 812ES was added. The mixture was stirred for a further hour at 90°C to give a polymeric composition (polymeric backbone). Meanwhile, 5.65g of Abitol was placed in the oven at 60°C for 15 mins. 0.01 g of 4-methoxyphenol and 3.01g of 9-anthracene methanol was then added to the polymeric composition, and stirring was then continued for a further 30 mins.

The Abitol was then added and the reaction mixture stirred in a water-bath (90°C) for 30 mins.

The resultant prepolymer was then left overnight to cool and was found to be a golden liquid.

### Example 2

### Preparation of Film for Peel Strength Testing

The prepolymer of Example 1 was mobilised in carbon tetrachloride (or by heating) and spread to a depth of 0.1mm (0.010 inches) thickness on flat Melinex film and left to dry at room temperature in the dark.

Once dry the prepolymer films on Melinex were laminated to release paper and UV irradiated for 20 minutes in a UV cabinet (wavelength greater than 300nm) through the clear Melinex layer. The resultant polymer was then prepared for peel strength testing.

Strips of the adhesive on Melinex having a width of 25mm were applied to standard steel plates using light finger pressure, followed by two passes of a 2kg roller (width 17.55mm (1¾ inch), diameter 40mm (4 inch)). The strips were allowed to remain laminated to the steel plates for 5 mins at 23°C in a 50% relative humidity environment and were then peeled off the steel at a rate of 300mm min⁻¹ along the length of the strips and using a 180°C peel test as described in British Pharmcopoeia Appendix XXH (Test 2).

The results were as follows:

| **Sample** | **Peel Strength (N/m)** | **Average (N/m)** |
|---|---|---|
| 1 | 1061 | |
| 2 | 1015 | 1050 |
| 3 | 1074 | |

### Example 3

Using the method of Example 1 the following linear and cross-linked dimerisable polyurethane prepolymers were produced.

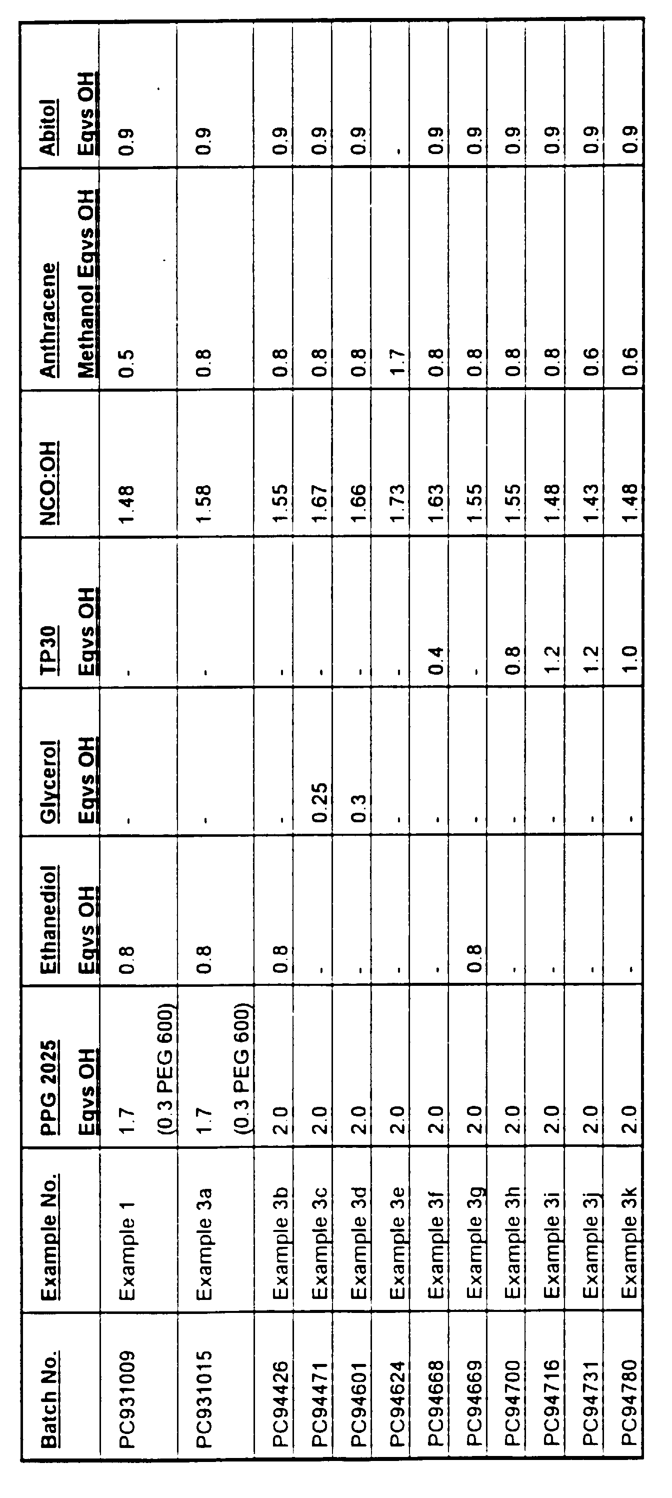

### Example 4

### Synthesis of Low Molecular Weight Polyacrylate Backbone Polymers

The molecular weight of the polyacrylates were varied by using different concentrations of the initiator bicyclohexyl peroxydicarbonate (BCHPC). The formulations for the polymeric backbone using n-butyl acrylate, 2-ethylhexyl acrylate and acrylic acid are given in Table 1.

**Table 1**

| **Sample** | **wt AA (g)** | **wt n-BA (g)** | **wt 2-EHA** | **% BCHPC** | **Batch No.** |
|---|---|---|---|---|---|
| No.8 A | 4.0 | 23.0 | 23.0 | 2 | PC941658 |
| No.8 B | 8.0 | 46.0 | 46.0 | 3 | PC941726 |
| No.8 C | 1.0 | 24.5 | 24.5 | 2 | PC941976 |

The syntheses were carried out using the same general procedure. A 250 ml flange flask was charged with the acrylic acid (AA), n-butyl acrylate (n-BA) and 2-ethyl hexyl acrylate (2-EHA) along with approximately 75 g of ethyl acetate solvent. The monomer solution was then stirred under a nitrogen atmosphere at 78°C until the mixture was homogeneous. Sample No.8 C was reacted at 60°C, as the solutions at higher temperature reacted very vigorously when the initiator was added.

Once the mixtures were homogeneous, the BCHPC was added in 40 - 50 ml of ethyl acetate as a single aliquot and then left to react for approximately four hours. Irganox 1010 antioxidant (1 %) was then added, again in 50 ml ethyl acetate, and the reaction solution left for a further 30 minutes before being batched. Once the solvent had been removed by rotary evaporation, the polyacrylate backbone polymers flowed at room temperature or when warmed (No.8 C). The molecular weights of these samples were determined by gel permeation chromatography and the results are given in Table 2. The polyacrylate backbone polymers are generally known as No.8 adhesives. The data for commercial No.8 adhesive is also shown for comparison. Although all three prepolymers have significantly lower molecular weights than commercial No. 8 adhesive, only No.8 A and B flowed at room temperature with the molecular weight of No.8 C being at the high end of acceptability in terms of its flow characteristics.

**Table 2**

| **Sample** | **Batch No.** | **Mp** | **Mn** | **Mw** | **Mz** | **PD** |
|---|---|---|---|---|---|---|
| Commercial No.8 | 218031 | 281,300 | 43,200 | 1,124,500 | 4,583,200 | 26.04 |
| No.8 A | PC941658 | 47,400 | 8,500 | 92,500 | 337,150 | 10.86 |
| No.8 B | PC941726 | 42,350 | 14,000 | 132,600 | 622,950 | 9.47 |
| No.8 C | PC941976 | 134.400 | 16.950 | 287,200 | 1,117,350 | 16.96 |

Acid values for No.8 A and B were determined to ensure all 8% acrylic acid had been incorporated into the polyacrylate backbone polymer (Table 3), and that the pendant carboxylic acid groups were free for esterification with anthracenemethanol. The results show that the experimental values correlate well with the theoretical acid value for 8% acrylic acid incorporation (gpc traces suggested no unincorporated acrylate monomers)

**Table 3**

| **Sample** | **Batch No** | **Acid Value (mg KOH/g)** | **Theoretical Acid Value 8% AA (mg KOH/g)** |
|---|---|---|---|
| No.8 A | PC941658 | 62.23 | 62.22 |
| No.8 B | PC941726 | 58.80 | 62.22 |

### Example 5

### Esterification of Polyacrylate Backbone Polymers (Synthesis of Polyacrylate Prepolymers)

The method used for esterification of the carboxylic acid groups pendant to the polymeric backbone chain employed a dehydrating agent, dicyclohexylcarbodiimide (DCC). This was used in conjunction with a dimethylaminopyridine (DMAP) catalyst with the reaction proceeding at room temperature. The following general procedure was initially used for the esterification reaction. For Example 5a, 6.09 g of No.8 prepolymer (containing 0.0068 moles acrylic acid) was placed in a dry jar along with 1.41 g of anthracenemethanol (0.0068 moles), 1.39 g DCC (0.0068 moles) and 0.33 g DMAP (0.0027 moles). Approximately 100 ml of anhydrous tetrahydrofuran (THF) was added to the reaction mixture which was left stirring overnight (∼ 24 hours). A precipitate was formed as a by-product of the esterification dicyclohexylurea (DHU) which was insoluble in THF and was therefore filtered from the prepolymer solution. The THF was then rotary evaporated leaving a viscous yellow liquid which was then spread thinly onto melinex for UV curing.

Using the abovementioned method the following polyacrylate prepolymers were produced.

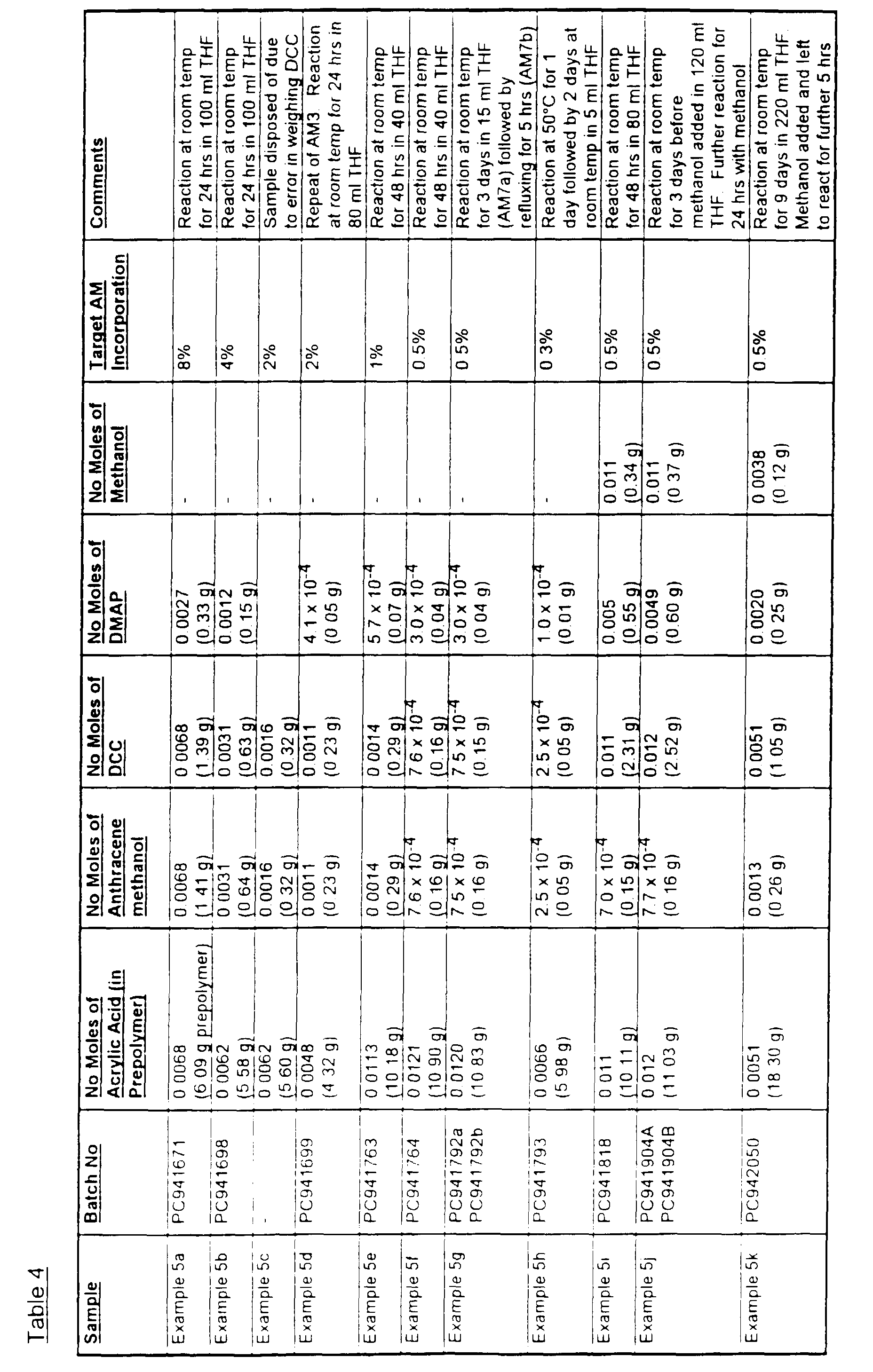

### Example 6

The level of anthracenemethanol incorporation into the prepolymer was measured by gpc using diode array detection (gpc-DAD). This showed the proportion of anthracenemethanol associated with the high molecular weight polyacrylate and the ureacted monomeric anthracenemethanol (Figure 1).

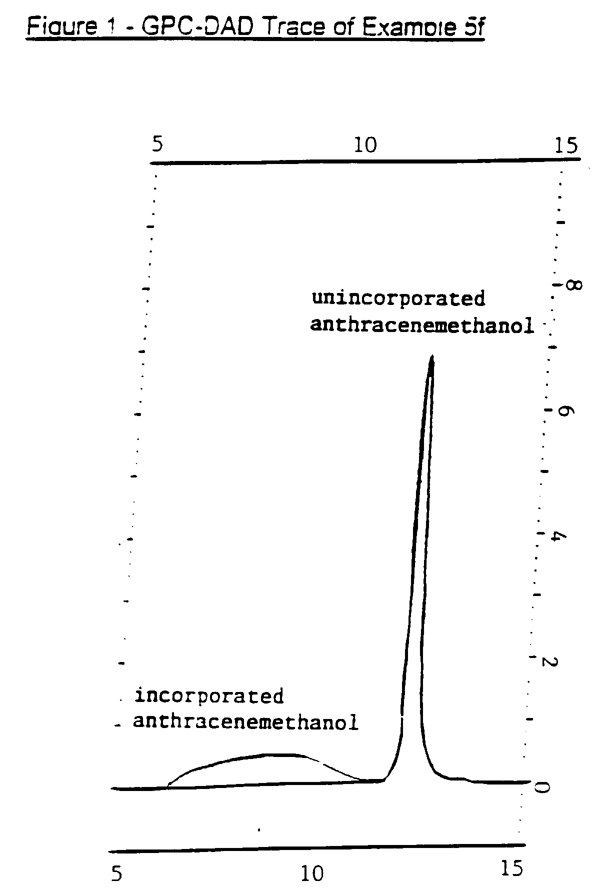

The level of anthracene methanol incorporated in the samples are illustrated in Table 5.

**Table 5**

| **Sample** | **Batch No** | **Actual AM Incorporation** | **Comments** |
|---|---|---|---|
| Example 5a | PC941671 | 1.95% | |
| Example 5b | PC941698 | 2.44% | |
| Example 5d | PC941699 | 1.14% | |
| Example 5e | PC941763 | 0.60% | |
| Example 5f | PC941764 | 0.13% | |
| Example 5gi | PC941792a | 0.16% - a | AM7b refluxed for 5 hrs after room temp reaction (AM7b) |
| Example 5gii | PC941792b | 0.20% - b | |
| Example 5h | PC941793 | 0.05% | |
| Example 5i | PC941818 | - | |
| Example 5jl | PC941904A | 0.41% - A | A - contains 0.09% unreacted AM B - precipitated into MeOH contains 0.05% unreacted AM |
| Example 5jii | PC941904B | 0.41% - B | |
| Example 5k | PC942050 | 0.25% | |

For the synthesis of samples Examples 5a to 5c, No.8 A was used, for Examples 5d to 5j, No.8 B was used, and for Example 5k, No.8 C (as defined in Example 4) was used. The target levels of anthracenemethanol incorporation, quoted in Table 4, correspond to the amount of acrylic acid to be reacted. In Example 5a a stoichiometric weight of anthracenemethanol was added to react with all 8% acrylic acid in the prepolymer. Therefore, for Example 5b only half of the acrylic acid was to be reacted resulting in 4% anthracenemethanol incorporation It should be noted that No.8 C was formulated with only 2% acrylic acid when calculating the level of anthracenemethanol in the prepolymer

The actual levels of incorporation, quoted in Table 5 were calculated from gpc-DAD traces and are based on the proportions of anthracenemethanol associated with the prepolymer and that remaining as monomer It was shown for Example 5j that precipitating the prepolymer in methanol after esterification resulted in the unreacted anthracenemethanol being washed from the sample. Therefore, the 0.41% incorporated into Example 5jii is the only anthracene available for dimerisation, whereas all other samples contained the unreacted anthracenemethanol blended in.

The favoured approach for the esterification reaction was to react any excess acrylic acid (remaining form the reaction with anthracenemethanol) with methanol, this being added after the anthracenemethanol had reacted.

### Example 7

The results of UV irradiating the acrylate prepolymers are summarised in Table 8.

**Table 8**

| **Sample** | **% Incorporated AM** | **Irradiation Time** | **Comment** |
|---|---|---|---|
| Example 5a | 1.95 | 12 mins | Low-tack film |
| | 1.95 | 20 mins | Non-tacky film |
| | 1.95 | 45 mins | Hard, non-tacky film |
| Example 5b | 2.44 | 30 mins | Non-tacky film |
| Example 5d | 1.14 | 30 mins | Low-tack film |
| | 1.14 | 1 hr | Non-tacky film |
| Example 5e | 0.60 | 1 hr | Slightly tacky film |
| Example 5f | 0.13 | 1 hr | Fairly good tack, high cohesive strength |
| Example 5i | - | 6.5 hrs | PSA with good tack and high cohesive strength |
| Example 5j | 0.41 | 1 hr | PSA with good tack and high cohesive strength |
| Example 5k | 0.25 | 1.5 hrs | PSA with subjectively good tack and cohesive strength. Peel strength of 25gsm spread - 267Nm⁻¹, clean peel from steel plates, no residue |

Although the level of incorporated anthracenemethanol is given in Table 8, the amount of unreacted, blended anthracenemethanol should also be considered when comparing results (see Table 5). However, a sample of No.8 B blended with anthracenemethanol produced a soft, low cohesive strength adhesive when UV irradiated for one hour. Therefore, this would indicate that the major effect on the curing of prepolymers is due to the dimerisation of bound anthracene rather than blended, as would be expected.

Prepolymers with levels of incorporated anthracene above 0.6% (AM1 - AM5) produced non-tacky films when UV irradiated. When the level of crosslinker was reduced to 0.13% (AM6) and 0.41% (AM10B), good PSAs with high cohesive strength were formed. Sample AM11 was the only one tested for peel strength. This adhesive peeled very cleanly from steel plates leaving no residue and failing adhesively.

## Claims

1. A pressure sensitive adhesive obtainable by photodimerising prepolymer molecules comprising a polymeric backbone capped with a dimerisable group.

2. An adhesive according to claim 1 wherein the polymeric backbone molecules are copolymers.

3. An adhesive according to claim 1 or 2 wherein the polymeric backbone molecules are polyacrylates with a number average molecular weight of 50,000 to 200,000.

4. An adhesive according to claim 1 or 2 wherein the polymeric backbone molecules are each a polyurethane derived from a polyester diol and a diisocyanate or a polyether diol and a diisocyanate, have a number average molecular weight of 3,000 to 15,000 and are chain extended by a chain extending agent.

5. An adhesive according to claim 1 wherein the dimer comprises groups selected from anthracene, cinnamyl, maleimide, coumarin or chalcone.

6. An adhesive according to claim 1 wherein the dimer comprises two anthracene moieties.

7. An adhesive according to claim 1 which comprises a cross-linked material.

8. A medical dressing comprising an adhesive according to claim 1.

9. A method for forming a pressure sensitive adhesive material, comprising using a prepolymer as described in claim 1.

10. A prepolymer as described in claim 1 which comprises a polyacrylate backbone capped with an anthracene alcohol group.

## Patentansprüche

1. Haftklebstoff erhältlich durch Photodimerisierung von Präpolymermolekülen mit einer Polymerkette, die mit einer dimerisierbaren Gruppe modifiziert ist.

2. Haftklebstoff nach Anspruch 1, wobei die Moleküle der Polymerkette Copolymere sind.

3. Haftklebstoff nach Anspruch 1 oder 2, wobei die Moleküle der Polymerkette Polyacrylate mit einem Zahlenmittel des Molekulargewichts von 50000 bis 200000 sind.

4. Haftklebstoff nach Anspruch 1 oder 2, wobei die Moleküle der Polymerkette jeweils ein Polyurethan sind, das aus einem Polyesterdiol und einem Diisocyanat oder einem Polyetherdiol und einem Diisocyanat hergestellt ist, und das ein Zahlenmittel des Molekulargewichts von 3000 bis 15000 hat und mittels eines Kettenverlängerers kettenverlängert ist.

5. Haftklebstoff nach Anspruch 1, wobei das Dimer Gruppen umfaßt, die aus Anthracen, Cinnamyl, Maleimid, Cumarin oder Chalkon ausgewählt sind.

6. Haftklebstoff nach Anspruch 1, wobei das Dimer zwei Anthraceneinheiten aufweist.

7. Haftklebstoff nach Anspruch 1, umfassend ein vernetztes Material.

8. Medizinisches Verbandmaterial mit einem Haftklebstoff nach Anspruch 1.

9. Verfahren zur Herstellung eines Haftklebematerials, umfassend die Verwendung eines in Anspruch 1 beschriebenen Präpolymers.

10. Präpolymer wie in Anspruch 1 beschrieben, umfassend eine mit einer Anthracenalkoholgruppe modifizierte Polyacrylatkette.

## Revendications

1. Adhésif sensible à la pression qui peut être obtenu par photodimérisation de molécules de prépolymère comprenant une chaîne principale de polymère coiffée par un groupe dimérisable.

2. Adhésif selon la revendication 1, dans lequel les molécules de la chaîne principale de polymère sont des copolymères.

3. Adhésif selon la revendication 1 ou 2, dans lequel les molécules de la chaîne principale de polymère sont des polyacrylates ayant une valeur de poids moléculaire moyen de 50 000 à 200 000.

4. Adhésif selon la revendication 1 ou 2, dans lequel les molécules de la chaîne principale de polymère sont chacune un polyuréthane dérivé d'un polyester diol et d'un diisocyanate ou d'un polyéther diol et d'un diisocyanate, elles ont une valeur de poids moléculaire moyen de 3 000 à 15 000 et elles sont à chaîne allongée par un agent d'extension de chaîne.

5. Adhésif selon la revendication 1, dans lequel le dimère comprend des groupes choisis parmi anthracène, cinnamyle, maléimide, coumarine ou chalcone.

6. Adhésif selon la revendication 1, dans lequel le dimère comprend deux radicaux anthracène.

7. Adhésif selon la revendication 1, qui comprend une matière réticulée.

8. Pansement médical comprenant un adhésif selon la revendication 1.

9. Procédé de préparation d'une matière adhésive sensible à la pression, comprenant l'utilisation d'un prépolymère comme décrit dans la revendication 1.

10. Prépolymère selon la revendication 1, qui comprend une chaîne principale de polyacrylate coiffée avec un groupe alcool d'anthracène.
